(19) **Europäisches Patentamt**
**European Patent Office**
**Office européen des brevets**

(11) **EP 3 906 934 B1**

(12) **EUROPEAN PATENT SPECIFICATION**

(45) Date of publication and mention of the grant of the patent:
**25.09.2024 Bulletin 2024/39**

(21) Application number: **20173355.7**

(22) Date of filing: **06.05.2020**

(51) International Patent Classification (IPC):
*A61K 38/08* (2019.01)      *C07K 7/06* (2006.01)
*A61K 47/10* (2017.01)      *A61K 47/18* (2017.01)
*A61K 9/00* (2006.01)       *A61P 31/12* (2006.01)
*A61P 31/16* (2006.01)

(52) Cooperative Patent Classification (CPC):
**A61P 31/16; A61K 9/0043; A61K 9/0056; A61K 9/0078; A61K 38/08; A61K 47/10; A61K 47/183; A61P 31/12;** C07K 7/06

(54) **APPLICATION OF DALARGIN FOR THE PREVENTION OF VIRAL RESPIRATORY INFECTIONS AND PREVENTION OF THE DEVELOPMENT OF COMPLICATIONS DURING VIRAL RESPIRATORY INFECTIONS**

VERWENDUNG VON DALARGIN ZUR PRÄVENTION VON VIRALEN ATEMWEGINFEKTIONEN UND ZUR PRÄVENTION DER ENTWICKLUNG VON KOMPLIKATIONEN WÄHREND VIRALEN ATEMWEGINFEKTIONEN

APPLICATION DE DALARGINE POUR LA PRÉVENTION D'INFECTIONS VIRALES DES VOIES RESPIRATOIRES ET LA PRÉVENTION DU DÉVELOPPEMENT DE COMPLICATIONS LORS D'INFECTIONS VIRALES DES VOIES RESPIRATOIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**10.11.2021 Bulletin 2021/45**

(73) Proprietor: **PVP Labs PTE. Ltd.**
**The Adelphi, Singapore 179803 (SG)**

(72) Inventors:
• **Chertorizhsky, Evgeny Alexandrovich**
**249034 Obninsk (RU)**
• **KOSORUKOV, Vyacheslav Stanislavovich**
**142152 Podolsk, Fedyukovo village (RU)**
• **KLEIMENOV, Aleksey Viktorovich**
**249192 Zhukov, Kaluga region (RU)**
• **MELNIKOV, Gennady Dmitrievich**
**117405 Moscow (RU)**

(74) Representative: **Spengler, Robert et al**
**Potthast & Spengler**
**Patentanwälte PartG mbB**
**Magirus-Deutz-Straße 12**
**89077 Ulm (DE)**

(56) References cited:
**EP-A1- 3 556 377      RU-C1- 2 436 588**
**SU-A1- 1 475 670**

• **OZBEJ ZUPANCIC ET AL: "Development and in vitro characterization of self-emulsifying drug delivery system (SEDDS) for oral opioid peptide delivery", JOURNAL DRUG DEVELOPMENT AND INDUSTRIAL PHARMACY, vol. 43, no. 10, 4 July 2017 (2017-07-04), US, pages 1694 - 1702, XP055727742, ISSN: 0363-9045, DOI: 10.1080/03639045.2017.1338722**

Remarks:
The file contains technical information submitted after the application was filed and not included in this specification

**Description**

**Pertinent art**

The invention is related to pharmaceutics and medicine and concerns the application of Dalargin solution in spray form for the irrigation of the oral cavity and/or nasopharynx in the form of inhalations using a nebulizer for the prevention of VRIs and/or for the prevention of their complications, especially in the early stages of the disease.

**State of the art**

Viral respiratory infections (VRIs) are currently the most common type of infections, accounting for over 90% of all infectious diseases [1, 2]. Influenza epidemics, during which approximately 5 to 15% of the population become infected, occur almost yearly. According to the WHO, approximately 1 billion people contract influenza every year, 3-5 million severe cases, and 290000 to 650000 influenza- related respiratory deaths worldwide. The social burden of VRIs is mostly characterised by high indirect costs, such as costs for the period of workplace absence, economic losses due to reduced labour productivity, and costs of workplace absence of family members taking care of the patient (economic losses in the USA due to temporary temporary disability only are evaluated at 232 million dollars) [3, 2]. Globally, an average of approximately 14.6 billion USD are spent on treating patients with VRIs and influenza.

For Russia this problem is extremely important due to its geographical and climatic characteristics. 27.3 to 41.2 million cases of respiratory infections are recorded in Russia yearly. VRIs have a high share in the overall morbidity structure: they account for almost 40% of days of disability [2, 4]. In particular, influenza is characterized by comparatively frequent development of severe complications, frequent involvement of the lower respiratory tract with progressive pneumonia and acute respiratory distress syndrome (ARDS) in children, as well as in young, middle-aged and elderly patients. For instance, the main problem among hospitalized patients with influenza A/H1N1/09 in almost all countries was progressive acute respiratory failure: pneumonia was diagnosed in 40-100% of patients, and ARDS, in 10-56% of patients [5]. Fatality rate for various types of VRI may be between 0.05% and 5%, or even more for individual pathogens.

These figures indicate the high burden of various types of VRI, as well as their serious social and economic consequences on a global scale. They convincingly show that it is important to protecting the population during seasonal VRI epidemics and necessary to have antiviral treatments suitable for the widest possible range of viral pathogens. Therefore, prevention of spread of this group of diseases is an important goal for any country.

VRIs are notable due to a wide range of viruses that cause influenza-like illnesses, which include over 200 diagnosable species. The most common pathogens include rhinoviruses, influenza viruses, parainfluenza viruses, respiratory syncytial virus (RSV), coronaviruses, metapneumoviruses, bocaviruses and adenoviruses [2, 6].

VRIs can often be severe, especially in patients with a compromised immune system or comorbidities. Manifestations of severe VRIs include pneumonia, particularly in the form of acute respiratory distress syndrome. [7].

VRIs of different types are associated with exacerbations of chronic diseases; additionally, VRIs may entail a wide range of sequelae, such as:

Pulmonary complications (pneumonia, bronchitis, ARDS);

Upper respiratory tract and ENT complications (otitis, sinusitis, rhinitis and tracheitis);

Cardiovascular complications (myocarditis, pericarditis);

Nervous system complications (meningitis, meningoencephalitis, encephalitis, neuralgia and polyradiculoneuritis);

secondary infections;

acute renal failure, 14 % to 53 %;

septic shock, 4 % to 8 %;

cardiomyopathy, including critical cardiomyopathy in 33 % of cases;

gastrointestinal tract involvement.

The development of complications usually profoundly changes and greatly complicates the therapy of the disease. For instance, mortality among patients with severe coronavirus disease 2019 (COVID-19) who require mechanical ventilation is as high as 70-80%, although at admission the disease may be mild or moderate, without serious lung involvement. The efficacy of pneumonia treatment using existing methods and protocols is limited. For this reason, the most appropriate way to fight this disease would involve preventing complications at the earliest stages of the disease or preventing infection as such.

In order to prevent complications and fatalities, it is extremely important to carry out antiviral therapy in order to eliminate the possibility of infection and development of disease, as well as take prophylactic measures to prevent the progression of the disease towards the development of all types of complications.

The following approaches are currently used in the prevention of viral diseases.

a) Vaccines. In standard practice of VRI prevention, this approach is almost exclusively employed against influenza. For most other types of VRI-causing viruses, vaccination makes little sense, since a certain level of immunity exists since childhood, the disease is mild, or immunogenic protective vaccines as such are non-existent. Polyvalent influenza vaccines that are used for prophylactic vaccination are based on WHO recommendations concerning influenza serotypes that are anticipated to be active in the coming season. The efficacy of influenza vaccines is 75-90 %.

Vaccination is especially important for people at risk of complications and for those sharing living space with or taking care of at-risk individuals.

On the other hand, vaccination as a method of preventing morbidity, and influenza in particular, has a number of drawbacks:

- Vaccination does not give an absolute guarantee that the disease will be prevented, that the type of influenza in the given year will match the predicted type; meanwhile, influenza vaccines are only effective for 1 year.

- Influenza vaccination is not for everyone. It is contraindicated to those with allergies to chicken proteins or a history of allergic reactions to influenza vaccines.

- In case of an ongoing influenza epidemic or pandemic there is often not enough time to develop strong immunity in response to vaccination, which substantially reduces the effect of vaccination in the population.

- Vaccination as a method of preventing VRIs caused by the wider range of pathogens is less effective, as no vaccine is available against the majority of pathogens, or it quickly becomes irrelevant due to the high variability of viral core proteins.

b) Immunomodulators. Another approach to VRI prophylaxis is based on administering various immunomodulators. They include thymus products, interleukins, interferons, biologically active peptides, polysaccharides of certain fungi, and therapeutic vaccines. Their activity is based on the ability to alter cell and tissue metabolism and activate immunocompetent cells. This pharmacological group is extremely diverse and includes several subgroups: interleukins, interferons, interferon inductors and other immunomodulators.

Interleukins and interferons are biotechnologically manufactured products based on endogenous proteins: human leukocyte interferon, Egiferon, products from the group of interferons alpha (Reaferon, Roferon, Intron A, Inrec, Viferon, Realdiron, Berofor, Egiferon, Velferon), interferons beta (Betaseron, Fron, Betaferon, Rebif, Bioferon, Avonex), and interferons gamma. The range of applications of these products is rather wide: they are effective against influenza viruses and viruses causing other respiratory diseases, hepatitis B and C viruses, CMV, measles virus, and AIDS virus. In some cases, this group of products is quite effective. However, disadvantages often include highly variable response and a large number of side effects, which may include fever, headache, myalgia, arthralgia, anaphylaxis, low blood pressure, tachycardia, etc.

Interleukins and interferons must be administered under medical supervision and cannot be used as a prophylactic intervention.

Drugs used in VRI prophylaxis include immunotropic products from groups such as "interferon inductors" and "other immunomodulators", which usually exhibit immunostimulant and immunomodulating effects. Oral medications include Kagocel, Arbidol, Ingavirin, Amixin and Groprinosin. Injectable products include thymus extracts (Thymoptin, Tactivin, Thymalin), Polyoxidonium, Thymogen, Immunofan, Neovir and Cycloferon.

Positive effect of many such medicines can be attributed to enhancement of the overall resistance of the body and its non-specific immunity, or activation of specific types of immune cells, as well as to activation of specific immune responses. These products are positioned as prophylactic and antiviral drugs. However, the efficacy of the majority of immunotropic products is relatively low, and products of this group failed to significantly exceed the threshold value of 30% in valid animal experiments investigating their protective effect on animal models of lethal viral infections, in contrast with specific products, such as influenza neuraminidase inhibitors, which feature protective indices of 70% and above. Additionally, efficacies they exhibit in clinical trials are not substantially higher than those in control groups, either untreated or treated using standard therapies, which draws plenty of criticism.

Chemical antiviral products against the influenza virus, such as neuraminidase inhibitors oseltamivir (Tamiflu) and zanamivir (Relenza), are not used for prophylaxis due to side effects.

An important point in the dynamics of disease development is the transition from the phase of infection, when the viral pathogen becomes firmly embedded in throat mucosa, to the descent of the disease to the bronchi and trachea and the development of a severe form of the disease. The first clinical manifestations of the disease, such as cough, fever, rhinitis, etc. emerge during this period. As a rule, VRI transition to a severe form is predictive of a low chance of efficient healing without long-term lung damage and involvement of other organs. Patients with a severe form of the disease naturally face the highest risk of death. Several approaches are used for preventing the development of severe complications of VRIs:

Antiviral drugs, in particular neuraminidase inhibitors oseltamivir (Tamiflu) and zanamivir (Relenza) in the case of the influenza virus. Considering frequent secondary involvement of bacterial flora, antibacterial therapy is administered, and the choice of the antibiotic being informed by the patient's condition [8].

Solutions of medicinal products delivered through a nebulizer, such as inhaled bronchodilators and anti-inflammatory products, are used for exerting a local effect directly on lung tissue [9].

However, these treatment methods have their own limitations. Targeted antivirals are chemical agents that specifically inhibit particular stages of viral replication and usually have a rather narrow spectrum. Anti-inflammatory and bronchodilator drugs improve patients' quality of life but mostly offer symptomatic treatment only, and don't meaningfully affect the underlying cause: viral load and concomitant bacterial complications.

Administration of Dalargin, an immunotropic product, as nasal solution is known as a treatment of VRIs [23] and as a pneumonia treatment administered by injection or infusion [10]. OZBEJ ZUPANCIC ET AL: IN "Development and in vitro characterization of self-emulsifying drug delivery system (SEDDS) for oral opioid peptide delivery", JOURNAL DRUG DEVELOPMENT AND INDUSTRIAL PHARMACY, vol. 43, no. 10,4 July 2017 (2017-07-04), pages 1694-1702, describe an oral administration of dalargin incorporated in a self-emulsifying drug delivery system.

Dalargin was approved for clinical use for the treatment of peptic ulcer disease during the exacerbation phase, acute pancreatitis, acute necrotizing pancreatitis, obliterative arterial disease of the lower limbs as part of combination therapy, and alcoholism. Dosage: a single dose for IV infusion during the treatment of acute necrotizing pancreatitis corresponds to 5 mg of Dalargin. Infusions are carried out 3-4 times daily at equal intervals. The course of treatment is 2 to 6 days. For example, SU 1 475 670 A1, and RU 2 436 588 C1 describe the use of dalargin for treating duodenal ulcer and/or pancreatitis.

Dalargin has a wide safety window and is a low-toxicity substance, with an $LD_{50}$ of 2.5 g/kg. Application to skin or mucosae at any concentration does not produce local irritation.

Dalargin also improves microcirculation and lymph drainage in the affected area [11, 12] and exhibits a direct antiviral and immunostimulant effect [23].

The closest analogue of the claimed invention is the application of a nasal medicinal composition containing the hexapeptide tyrosyl-D-alanyl-glycyl-phenylalanyl-leucyl-arginine or its pharmaceutically acceptable salt for the treatment of viral respiratory infections (VRIs), as disclosed by EP 3 556 377 A1 (Patent RU2672888). The hexapeptide was demonstrated to exhibit a direct antiviral effect. Application of the proposed composition in the form of a nasal spray allows to use it as monotherapy or in combination with other medicinal products as a product for the treatment and prevention of VRIs, in particular, influenza. However, the described method of application of the hexapeptide involves the irrigation of the nasal passages only. This does not allow to create an antiviral barrier effect across the entire oral, oropharyngeal and nasopharyngeal mucosa and completely block the "portal of entry". Additionally, in order to ensure storage stability during use, the composition must contain a microbial growth inhibitor, such as benzalkonium chloride, usually in the concentration range from 0.005% to 0.1%. In addition to bactericidal effect, which allows for long-term storage of vials of the solution, benzalkonium chloride, a component of the formulation, exhibits a substantial antibacterial and antiviral local effect with respect to influenza viruses and coronaviruses when the formulation is used in an inhaled form or as spray [24].

To summarize the above, we can state that the currently available publications do not describe the application of the active ingredient, the hexapeptide Dalargin, as a product for creating the effect of a barrier to the ingress of viral infection across the entire surface of the oral cavity, nasopharynx and oropharynx, or its use as the active component preventing the development of complications of acute respiratory infections, in particular, those of unclear origin. The use of the inhaled form of the product for these purposes is also not described.

The goal of this invention is to expand the range of applications of tyrosyl-D-alanyl-glycyl-phenylalanyl-leucyl-arginine diacetate, hereinafter referred to as Dalargin, in the prevention of contracting acute respiratory infections of viral origin and in the prevention of complications.

In order to solve this problem, we propose to use Dalargin in spray form and/or in an inhaled form for the prevention of VRIs or for the prevention of their complications. The problem is solved by Tyrosyl-D-alanyl-glycyl-phenylalanyl-leucyl-arginine diacetate for use in spray form and/or in an inhaled form for the prevention of viral respiratory infections or for the prevention of their complications by administration in spray form to irrigate the nasal passages, nasopharynx, oropharynx and the oral cavity and/or by inhalation.

In a particular embodiment of the invention, Dalargin is used as a component of a composition that is administered as a spray and/or as an inhalation solution.

In a particular embodiment of the invention, Dalargin solution is used for the prevention of VRIs via inhalation and/or as a spray with a particle size of 5-50 $\mu$m.

In a particular embodiment of the invention for the protection against contracting VRIs, Dalargin solution is used for irrigating the entire surface of the nasopharynx, oropharynx, oral cavity and is applied sublingually, under the tongue, to create a protective barrier effect on mucous membranes.

In a particular embodiment of the invention, Dalargin solution is used for the prevention of VRI complications via inhalation with a particle size of 0.5 - 10 $\mu$m, preferably 0.5 - 3 $\mu$m.

In a particular embodiment of the invention, the hexapeptide-containing composition which is administered in spray form and/or as an inhalation solution contains a microbial growth inhibitor, such as benzalkonium chloride, usually in the concentration range of 0.005-0.1 wt %, preferably 0.01%.

In a particular embodiment of the invention, the composition has an acidic pH in the range from 3 to 7, preferably pH = 4.0-5.5.

In a particular embodiment of the invention, the hexapeptide-containing composition, which is administered in spray form and/or as an inhalation solution, contains acetate buffer.

Technical results of the claimed invention are:

- expansion of the technical toolkit of products for inhalation therapy for the prophylaxis of VRIs or prevention of the development of complications;

- achievement of therapeutic concentrations directly in the tissues that are exposed to viruses, including the nasopharynx, larynx, trachea, bronchi, alveoli, which produces prompt and active local response in the form of heightened antiviral activity specifically at the site of infection.

- achievement of indirectly caused positive reactions, such as antitussive effect and reduced shortness of breath;

- achievement of stabilisation of patients with mild or moderate forms of VRI, in particular when burdened with complications of viral and/or bacterial origin and effects of local pathological hyperactivation of immune response;

- the product demonstrated high efficacy against different types of viruses, which was comparable to specific targeted antiviral drugs, such as Oseltamivir, in animal tests;

- particle size of the aerosolised pharmaceutical substance vary over a wide range, from 0.5 $\mu$m to 50 $\mu$m, which produces both a prophylactic protective effect (irrigation of the upper respiratory tract is sufficient for prophylaxis; the associated particle size is relatively large: 5-50 $\mu$m) and a therapeutic effect (prevention of complications necessitates acting upon deeper regions, which are accessible to finer particles, 0.5-3 $\mu$m).

- as a recommended inhaled dose, we propose to use a solution of 0.005-0.5% of Dalargin in buffer solution with a pH in the range from 3 to 7, preferably pH = 4.0-5.5, a single dose being 0.5 to 50 mg in a composition with acetate buffer. It was demonstrated that the antiviral and immunotropic efficacy of the spray form and inhalation form of Dalargin is significantly reduced in solutions with pH above 6 or with buffers other than acetate.

Advantages of the inhalation form of Dalargin include its highly specific effect on the target organ and vastly extended duration of effect when administered through the lungs relative to systemic administration. When administered by injection or infusion, the mean elimination half-life of Dalargin is 12 minutes [13]. Elimination occurs by rapid degradation of the peptide in the liver and bloodstream. The inhaled form, despite substantial losses associated with this mode of administration, remains in the active form on the surface of the tracheal and bronchial mucosa for a much longer time and exhibits a direct therapeutic effect.

As Dalargin is a peptide, after administration it is rapidly degraded by peptidases to oligopeptides and amino acids, which are then reused in the synthesis of the organism's own proteins [14]. In this case, after a Dalargin product is administered systemically, it is rapidly degraded, and its efficacy in target organs declines. Administration of the inhaled dosage form allows maximum working concentration of the product to be achieved specifically in the target organ and maintained over a long period. If the active substance is delivered through the bronchial and throat mucosa and lung tissues, it acts on the local populations of immune cells, such as leukocytes and macrophages.

The process of degradation of the peptide Dalargin (Tyr-D-Ala-Gly-Phe-Leu-Arg) involves cleavage of the C-terminal amino acid, which yields the five-member peptide Tyr-D-Ala-Gly-Phe-Leu with a considerable affinity to delta receptors. The next metabolite, the tetrapeptide Tyr-D-Ala-Gly-Phe, exhibits affinity to mu receptors. Mu and delta receptors are found in the cough centre of the medulla, and systemic application of antagonists of these receptors inhibits the cough reflex. This usually also affects other organs and zones of the brain that contain opioid receptors. Additionally, since receptors of this group are found in peripheral neural pathways, Dalargin can also affect the conduction of signals along these neural pathways. [15] Dalargin inhalation mostly produces local effects of short-lived metabolites of the peptide on peripheral opioid receptors, which manifests itself in reduced response of receptor cells to stimulation and reduced intensity of the signal transmitted to the cough centre. This results in reduced coughing severity for 2-4 hours, for as long as Dalargin remains in the mucous tissues of the lung at a particular concentration. This effect does not involve the respiratory centre. A certain local analgesic effect also improves the overall condition of the patient. Indeed, clinical testing showed that patients reported substantial improvement of breathing and alleviation or elimination of cough, usually dry cough. This, in turn, alleviates the irritation of the respiratory tract, reduces the loss of the inhaled product due to coughing and improves the patients' quality of life.

Shortness of breath is one of the most common symptoms of VRIs. The patient is often unable to perform the most basic actions without experiencing shortness of breath, panting, and a sensation of lacking air. Descriptions of various methods of the treatment of shortness of breath report positive effects of systemic administration of certain opioid receptor agonists. A variety of opioids administered in inhaled form showed a positive effect in most studies [16]. Systemically administered opioids are also recommended for treating shortness of breath in such patients, but side effects may limit their use. Despite the positive effect, uses of such therapies are severely limited due to typical side effects and restrictions on the sales of opioid drugs. Inhalation of Dalargin is additionally associated with improved quality of respiration. Investigational clinical use of the drug significantly reduced shortness of breath and was associated with "lightness of breathing". A presumed mechanism by which this effect might occur is based on short-term local effects of Dalargin metabolites, which to a certain degree exhibit properties of mu and delta receptor agonists. Unlike classical opioids, these metabolites are short-lived and only act locally, in the zone where the inhaled aerosol is deposited in the lungs, predominantly in the larynx, bronchi and trachea.

Therefore, Dalargin in an inhaled form or in spray form can exhibit a direct antiviral effect, helps alleviate coughing and reduce negative effects associated with shortness of breath in VRI patients, and prevents the development of complications and transition of the disease to a severe form; additionally, the use of Dalargin in spray form for simultaneous irrigation of the oral cavity, nasopharynx, oropharynx and the sublingual area offers significant protection against contracting VRIs.

The studies carried out by us demonstrated that the effects of Dalargin - in particular the direct antiviral effect, activation of cell-mediated immunity and the "barrier effect", ability to enhance the factors of local and systemic immunity, reduce cough and alleviate difficulty breathing - allow it to be used as an effective therapeutic product for the prevention of VRIs and their complications.

Dalargin activates immune and antistressor responses of the patient's organism, which are universal in nature and are triggered during viral infections independent of the specific pathogen.

Brief description of explanatory materials.

Table 1. Virus inhibiting properties of Dalargin with respect to influenza virus and human adenovirus in cell culture.

Table 2. Statistics of animal mortality due to lethal influenza pneumonia caused by influenza virus A/Puerto Rico/8/34 (H1N1). Comment: * Statistically significant differences from the control group ($p < 0.05$).

Table 3. Animal survival statistics in a model of lethal influenza pneumonia caused by influenza virus A/California/04/2009. Comment: ** Statistically significant differences from the active control group ($p < 0.05$) (Ingavirin).

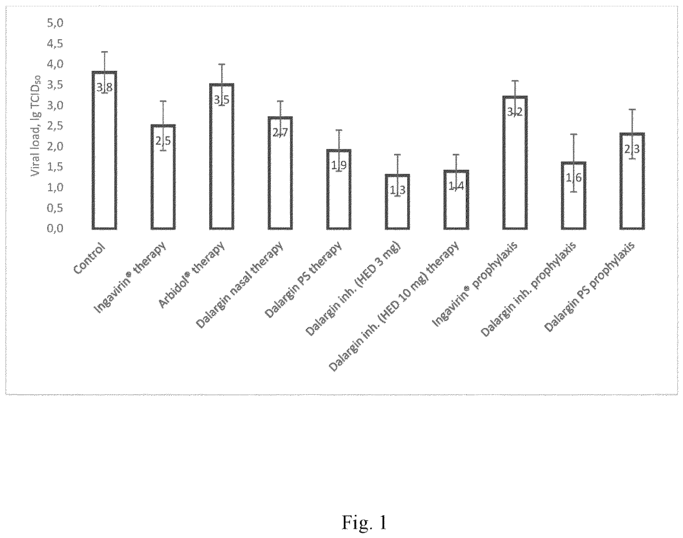

Fig. 1. Titre of influenza virus A/California/04/2009 in the lungs of infected mice 5 days after the challenge. Comment: error bars represent 95% confidence interval.

Disclosure of the technical character of the invention.

The inhaled form of the product can be prepared as a sterile solution with a concentration of 0.005-0.5% Dalargin in buffer solution, preferably acetate buffer, with a pH value of 3 to 8, preferably pH = 4.0-5.5, a single dose being 0.5 to 50 mg in 5-20 mL of the solution. Dalargin solution is administered through a compressor nebulizer (including models manufactured by Med2000, Omron, B.Well, etc.), the recommended size range of generated particles being 0.5-10 $\mu$m, preferably 0.5-3.0 $\mu$m. This specific particle size ensures the most complete delivery of the active ingredient to the middle and lower respiratory tract.

The product is delivered to the upper respiratory tract as a spray with a particle size of 5-50 $\mu$m.

Additional excipients may be added to the formulation of the solution, such as components of buffer salts, stabilizers, thickeners, pharmacologically acceptable surfactants, physiological salts and preservatives.

The preferred type of buffer salts is acetate buffer, which contains sodium acetate and acetic acid, with a preferred pH value of 4.0-5.5. Citrate buffer can also be used. Our studies showed that the low pH of the acetate-based buffer solution ensures 2-4 times higher specific antiviral and immunotropic activity of Dalargin when administered into the nose, nasopharynx, oropharynx, oral cavity or used sublingually for prophylactic or therapeutic and prophylactic purposes. This ensures a more stable effect when lower doses of the drug (1-4 mg per dose) are administered. Additionally, a physiologically active buffer on the basis of sodium hydrogen phosphate dihydrate and sodium dihydrogen phosphate dihydrate can be used; however, this buffer is not sufficiently efficient in the pH range from 4.0 to 5.5 in terms of its buffer capacity.

In order to ensure a sufficiently long shelf life of the solution in a spray dispenser and after the start of use, the solution should contain benzalkonium chloride, a bactericidal component, usually in the concentration range from 0.005% to 0.1%.

Components such as disodium edetate dihydrate (0.1-2 mg/mL) and sodium chloride (1-10 mg/mL) can also be added as stabilizers. These additives can potentially extend the shelf life of solutions and lyophilised powders containing the active component Dalargin. Additionally, sodium chloride is used as a component that gives the solution isotonic properties.

Solution viscosity may be important for the administration of Dalargin solution in spray form. Excipients such as microcrystalline cellulose, sodium carboxymethyl cellulose, macrogol, propylene glycol, glycerol, polyvinylpyrrolidone and N-methylpyrrolidone can be used as viscosity regulators.

Various polymers with bioadhesive properties, surfactants, or non-ionic detergents with mucoadhesive properties, such as poloxamers, can be used for improving the adhesion of the spray solution to mucous membranes.

For standard application in a nebulizer, the nebulizer chamber is charged with 5-10 mL of 0.02-0.2% Dalargin solution and connected to the compressor of a nebulizer. A mask or mouthpiece is attached to the chamber with the drug. Then a compressor is started, and the mask is firmly applied to the face to make sure that respiration occurs through the nose and mouth. If a mouthpiece is used, it is placed in the mouth and firmly held with the lips. Respiratory rate should be calm, inspiratory movements should be deep and gentle.

Inhalation is carried out for 2-15 minutes 2-3 times a day. The preferred dosage of the drug per one inhalation session can be from 1 to 6 mg, which is sufficient for the drug to exhibit its major effects. In case of therapy of moderate VRIs, the amount of the active ingredient may be as high as 50 mg with extended (up to 30 minutes) inhalation.

If the solution is used in spray form, the prepared solution is placed in a container equipped with a sprayer that produces 10-50 $\mu$m particles, nasal passages are irrigated with 1-2 puffs, and the oral cavity is irrigated with 2-3 puffs 2-3 times a day. The preferred concentration of the drug in the spray solution can be 0.05-0.5 % of Dalargin.

Embodiment of the invention.

**Example 1. Preparation of 0.05 % Dalargin inhalation solution.**

Dalargin inhalation solution is prepared in clean rooms. The optimum therapeutic concentration of Dalargin for inhalation for the purposes of prevention and control of complications at the initial stages of VRIs is 0.05%. In this case the optimum inhaled volume can be 4 to 10 mL, which corresponds to 2-5 mg of Dalargin per inhalation procedure.

The following procedure is used for the preparation of 10 L of the inhalation solution. Prepare an acetate buffer concentrate by mixing 490 mL of 0.2 sodium acetate solution and 510 mL of 0.2 M acetic acid. This is a 10-fold concentrate relative to the final solution, which must contain 0.005-0.05 M of acetate buffer, preferably 0.02 M. The pH value of the concentrate must be in the range from 4.3 to 4.8. If necessary, adjust the pH by adding 1 M sodium acetate solution or 1 M acetic acid. Place 1 L of the acetate buffer concentrate in a stirred reactor. Add 7 L of water for injections. Buffer solution can also be prepared on the basis of sodium citrate using a similar procedure, but acetate buffer is preferable.

In order to prepare 0.05 % Dalargin solution for inhalation, use 5000 mg of Dalargin lyophilisate powder. If the API is used in liquid form, take the amount of the API solution equivalent to 5000 mg of Dalargin dry powder. For example, in the case of 10% liquid API, take 50 mL of API solution. Mix the components in a stirred reactor until the components have completely dissolved. Monitor solution pH and adjust if necessary by adding 1 M sodium acetate solution or 1 M acetic acid. Bring up to 10 L and stop the mixing. Filter the resulting solution by dead-end sterile filtration and fill in 5 or 10 mL sterile vials, preferably of amber glass or opaque. This procedure, similarly to examples 2, 3 and 4 provided above, can be scaled to the required volume of Dalargin solution taking into account the particular features of the specific pharmaceutical production facility.

The resulting solution can be stored refrigerated for a long time and used for inhalation without additional manipulations. Transfer the required quantity of the solution from the vial to a nebulizer chamber and administer by inhalation.

**Example 2. Preparation of 0.2 % Dalargin inhalation solution.**

High doses of Dalargin can be necessary for preventing complications in patients with moderate VRIs. This necessitates preparing 0.2% Dalargin inhalation solution.

Prepare the buffer solution similarly to Example 1. In order to prepare 0.2 % Dalargin inhalation solution, use 20 000 mg of Dalargin lyophilisate powder. If 10% liquid API is used, take 200 mL. Sterilise the resulting solution and fill an adequate quantity (preferably 4-10 mL) in vials under aseptic conditions.

**Example 3. Preparation of Dalargin spray solution for irrigation of the mucous membranes of the nose and throat.**

Dalargin solution for the irrigation of the nasopharynx, oropharynx, oral cavity and the sublingual area has a Dalargin concentration of preferably 0.05-0.4%. As a rule, a solution manufactured according to the applicable quality standards, with pH controlled to the range of 4 to 5, does not require concentrations above 0.1-0.2% to exhibit sufficient activity.

The solution is prepared in clean rooms. In order to prepare 10 L of 0.1 % Dalargin solution for spray administration, prepare 0.02 M acetate buffer similarly to Example 1. Add 1 g of benzalkonium chloride to the reactor vessel in powder form or in the form of concentrated solution to achieve a concentration of 0.01% in the final solution. In order to prepare 0.1 % Dalargin solution for spray administration, use 10 000 mg of Dalargin lyophilisate powder. If 10% liquid API is used, take 100 mL. Monitor solution pH and, if necessary, adjust to pH = 4-5 by adding 1 M sodium acetate solution or 1 M acetic acid. Bring up to 10 L and stop the mixing. Filter the resulting solution by dead-end sterile filtration and fill in 5 or 10 mL sterile vials, preferably of amber glass or opaque, with a sprayer head.

**Example 4. Preparation of prolonged-action 0.3 % Dalargin spray solution for irrigation of the mucous membranes of the nose and throat.**

Spray solution according to Example 3 contains the minimum number of components for producing a specific protective antiviral effect. Addition of viscosity-enhancing components to the spray solution ensures longer retention of the solution on mucous membranes, gradual release of the solution and extended duration of the effect. This version of preparation of spray solution requires a lower dosing frequency in clinical practice.

In order to prepare 10 L of 0.3 % Dalargin solution for spray administration, prepare 0.02 M acetate buffer similarly to Example 1. Charge a reactor vessel with 1 g of benzalkonium chloride, 50 g of sodium chloride, 5 g of sodium edetate, 100 g of poloxamer 407, 600 g of polyethylene glycol 400, 600 g of propylene glycol, 30 000 mg of Dalargin or an equivalent quantity of concentrated API solution. Thoroughly mix the components in a stirred reactor until the components have completely dissolved, preferably using an evacuation procedure for degassing. Monitor solution pH and adjust if necessary by adding 1 M sodium acetate solution or 1 M acetic acid. Bring up to 10 L and stop the mixing. Filter the resulting solution by dead-end sterile filtration and fill in 5 or 10 mL sterile vials.

The resulting solution is characterised by a relatively viscous consistency that can effectively coat mucous membranes of the throat and oral cavity, ensuring gradual absorption of the active component of Dalargin in surface tissues and ensuring greatly increased efficacy relative to formulations without this complex of excipients.

**Example 5.**

**Antiviral activity of Dalargin was determined in vitro** with respect to influenza virus A/Puerto Rico/8/34 (H1N1) relative to Remantadin (at a dose of 5 $\mu$g/mL) and human adenovirus 5 relative to Aciclovir (at a dose of 30 $\mu$g/mL). Cells of the corresponding cell line (MDCK for influenza virus and Vero for adenovirus) were infected with the virus at a multiplicity of infection of 0.01 in the presence of Dalargin in the concentration range from 100 to 1 $\mu$g/mL Plates with the cells were incubated for 1 h, whereupon unbound virus was washed off, culture medium was replaced with a fresh one, which contained Dalargin at the same concentration, and the plates were incubated in a thermostat for 24 h. At the end of the incubation, the infectious virus titre was determined by titration on a permissive cell culture. To do this, ten-fold serial dilutions ($10^{-1}$-$10^{-7}$) of the culture fluid were prepared, these dilutions were used to infect cells of the respective line inoculated in 96-well plates, and the plates were incubated at 36 °C under a 5% $CO_2$ atmosphere for 72 h. At the end of this period, the presence of the virus in the wells was detected by the presence of a specific cytopathogenic effect (CPE) for adenovirus or by a positive hemagglutination assay (HA) in the culture fluid (influenza virus).

For a hemagglutination assay (HA), 100 $\mu$L of the culture fluid were transferred to the respective wells of an immunology microplate, followed by an equal volume of 1 % chicken red blood cells in saline. The plates were incubated at 20 °C for 1 h. At the end of this period, HA results were evaluated in the wells by visual inspection.

Test results for the antiviral activity of Dalargin against influenza virus and human adenovirus in cell cultures are provided in Table 1.

Differences with a p value below 0.05 were considered statistically significant.

The following parameters were calculated based on the collected data:

- 50 % cytotoxic concentration ($CC_{50}$), i.e. the concentration of the compound that reduces absorbance in wells by a factor of two relative to control cells;

- Titre units: virus titre unit was the negative decimal logarithm of the maximum dilution of the virus capable of producing a positive hemagglutination reaction (influenza virus) or specific CPE (adenovirus), i.e. the 50 % tissue culture infective dose of the virus (TCID□□);

- Virus titre was expressed in decimal logarithms of its concentration in the material in the appropriate titre units;

IC□□ is the 50 % effective (i.e. virus inhibiting) concentration of the substance;

SI, the selectivity index of the substance, is the ratio of the toxic concentration (CC□□) to the effective concentration (IC□□).

It follows from the results that model viruses effectively multiplied in the tissue cultures, and virus titres after 24 h of cultivation reached a value of 5.5 (influenza virus) or 4.5 (adenovirus) lg TCID□□/0.1 mL. Reference drugs Remantadin and Aciclovir did not exhibit antiviral properties, as the selected viruses were insensitive to these drugs.

At the same time, Dalargin resulted in a dose-dependent reduction of the infectious activity of both viruses. This effect was more pronounced for the influenza virus (IC□□ = 0.8 μg/mL, SI > 37.5) and somewhat less pronounced for adenovirus (IC□□ - 2.5 μg/mL, SI = 12).

Therefore, in vitro test results confirmed that Dalargin exhibits a virus inhibiting effect.

**Example 6. In vivo evaluation of the antiviral activity of Dalargin administered by inhalation as a therapeutic/preventive or purely preventive regimen in a model of influenza pneumonia in mice with respect to a relevant strain of influenza virus (A/Puerto Rico/8/34 (H1N1)).**

Description of the in vivo model. The study was carried out using 297 female Balb/c mice with an average body weight of 25 ± 4.6 g. Taking into account inter-species differences of respiration characteristics is especially important for the inhaled route of administration in animal models [14, 15, 16]. Despite the fact that a number of researchers suggest using direct intratracheal administration of drug solutions (mostly as part of toxicology studies), this approach is evidently not quite suitable for therapeutic models. When modelling the therapeutic effect of drugs administered by inhalation, a similar route of administration must be emulated in animals. However, in this case accurate determination and normalization of respiratory function is required for calculating the doses received by the animals.

The respiratory function undergoes dramatic changes depending on the physiological state (e.g. at rest or during stress), which invariably impacts its characteristics [16]. Additionally, different lines of mice also substantially differ in such parameters of the respiratory system as respiratory rate, respiratory volume and respiratory minute volume [15]. Moreover, the proportion of the drug reaching the lower respiratory tract must be taken into account, as it is the decisive factor of pharmacological activity; meanwhile, it depends on a number of parameters (such as aerosol particle size) and, consequently, on ventilation values used in the model.

The following formula was used in our experiment to calculate the dose absorbed in the lower respiratory tract (DoseAbsorb):

$$DoseAbsorb\,(\mathrm{mg/kg}) = \frac{ConcSol\left(\frac{\mathrm{mg}}{\mathrm{mL}}\right) * Vneb\left(\frac{\mathrm{mL}}{\mathrm{min}}\right) * time\,(\mathrm{min}) * RatioAbsorb * m(\mathrm{kg}) * V\left(\frac{\mathrm{L}}{\mathrm{kg}} * \mathrm{min}\right)}{Flow\left(\frac{\mathrm{L}}{\mathrm{min}}\right) * 1\,\mathrm{min}}$$

where ConcSol (mg/mL) is the concentration in solution, Vneb (mL/min) is the aerosol generation rate (0.3 mL/min) time (min) is the duration of animal exposure in the chamber, RatioAbsorb is alveolar retention (the proportion of aerosol particles deposited in the lungs; it is determined by particle size and was assumed equal to 7.3 % based on data obtained in preliminary tests [14]); m (kg) is the weight of the animals (25 g), V (L/kg·min) is the respiratory minute volume (1 L/kg·min); Flow (L/min) is the air flow rate through the chamber (17 L/min).

Materials and Methods. In order to administer inhalations, the mice were placed in an 14.5 L acrylic glass chamber, 18 animals to a chamber. The chamber was equipped with two running wheels to prevent the animals from huddling and allow them to move.

A SideStream nebulizer was used to convert the solutions to an inhaled form; the nebulizer produces 0.5-3 μm

particles at a pressure of 3 atmospheres and a flow rate of 17 L/min. The solution was aerosolised at a rate of 0.3 mL/min.

For inhalation treatment, Dalargin was dissolved in double-distilled water to a final concentration of 1 %, 3 % and 5 %. Doses for mice were calculated by back-conversion of the following human equivalent doses (HED): 1, 20 and 40 mg/day.

Dosing was controlled by adjusting the concentration of the inhalation solution and the duration of animal exposure in the chamber.

The animals were randomized across 9 groups of 33 animals each (30 animals for mortality evaluation and 3 animals for euthanasia on day 3):

Group 1 (control: inhalation of saline solution twice a day);

Group 2 (Tamiflu™ at a dose of 20 mg/kg once a day intragastrically);

Group 2 (Kagocel® at a dose of 12 mg/kg once a day intragastrically);

Group 4 (inhalation of 1 % Dalargin solution for 14 minutes twice a day). The dose received (the dose that reached the alveolae) was 0.36 mg/kg. The HED corresponds to 2 mg/kg;

Group 5: the mice received inhalations of 3 % Dalargin solution for 45 minutes twice a day. The received single dose (the dose that reached the alveolae) was 1.74 mg/kg. Daily EHD corresponds to 20 mg/kg;

Group 6: the mice received inhalations of 5 % Dalargin solution for 55 minutes twice a day. The received single dose (the dose that reached the alveolae) was 3.54 mg/kg. Daily EHD corresponds to 40 mg/kg.

Group 7: the mice received a prophylactic regimen of Kagocel® at a dose of 12 mg/kg intragastrically once a day on days 3, 2, 1 and at 1 hour before challenge.

Group 8: the mice received inhalations of 3 % Dalargin solution for 45 minutes twice a day on days 3, 2 and 1 and at 1 hour before challenge; no further treatment was administered after the challenge.

Group 9: the product was administered nasally and applied by spraying on the posterior pharyngeal wall (pharyngeal spray, PS) at a dose of 0.5 mg/kg (as 5 $\mu$L of 0.2 % solution and 10 $\mu$L of 0.1 % solution) twice a day on days 3, 2 and 1 and at 1 hour before challenge; no further treatment was administered after the challenge.

The animals were challenged under ether anaesthesia. Mice were challenged with $2.5 \times 10^3$ EID$\square\square$ per animal delivered as 0.025 mL of virus-containing material per nostril.

Reference products administered for prophylactic and therapeutic purposes were given intragastrically at 24 h (Kagocel®) and 1 h (Tamiflu™, Kagocel®) before challenge and for 5 days after challenge. Inhalations of Dalargin administered for prophylactic and therapeutic purposes (groups 3, 4 and 5) were given daily, once or twice a day for 6 days (24 h and 1 h before challenge and for 5 days after challenge). As a preventive treatment, group 7 received the comparator drug Kagocel® at a dose of 12 mg/kg intragastrically once a day on days 3, 2, 1 and at 1 hour before challenge. As a prophylactic regimen, group 8 received inhalations of 3 % Dalargin solution for 45 minutes twice a day, and group 9 received Dalargin nasally and as a spray applied to the posterior pharyngeal wall (pharyngeal spray, PS) at a dose of 0.5 mg/kg (as 5 $\mu$L of 0.2 % solution and 10 $\mu$L of 0.1 % solution) once a day on days 3, 2 and 1 and at 1 hour before challenge; no further treatment was administered after the challenge.

The following parameters were used as primary efficacy endpoints:

Mortality (%): ratio of the number of dead animals relative to the total number of infected animals in the group: M% = M / Nt, where M is the number of dead animals, and Nt is the number of infected animals.

Protective index (%): ratio of the difference in mortality percentage in the control and experimental groups to mortality percentage in the control group: PI = ((Me - Me) / Me) $\times$ 100 %, where Me is the control group mortality, and Me is the experimental group mortality.

Survival was evaluated using the Mantel-Cox method adjusted for multiple comparisons, Kaplan-Meier curves were plotted using IBM SPSS v. 22.0.

Results

Clinical signs of disease in infected mice were typical of influenza infections. They included difficulty breathing, ataxia, tremor and reduced consumption of feed and water. There was no non-specific mortality in the control group, which consisted of intact animals. The data on mortality statistics in the control and experimental groups are summarized in Table 2.

Three animals from each group (for a total of n = 6) were euthanised on days 3 and 12 of the experiment in order to evaluate histological features of lung tissue.

On days 3 after challenge, control animals developed pronounced interstitial oedema; observations also revealed areas of haemorrhagic oedema, neutrophilic infiltration, and collapsed alveolae with serous exudate in their lumina. These phenomena are indicative of acute respiratory distress syndrome. A similar pattern was observed in groups 3 and 7 and partially in group 9. Comparator drugs failed to demonstrate a significant effect on histological features. These changes were far less pronounced in the groups that received Dalargin. In groups 8 and 9, in which Dalargin was used preventively, some of the animals showed no signs of pathology and did not exhibit the characteristic histological features of infection.

On day 12, surviving animals in the control group exhibited hyperplasia of bronchial epithelium, cellular exudate in the bronchial lumen and intense cellular infiltration. These fibrotic changes frequently underlie pulmonary fibrosis, a common complication of influenza. The use of comparator drugs was associated with less pronounced inflammatory changes. One half of the animals receiving Dalargin exhibited no signs of pathology in the histological features of pulmonary tissue, indicating that the drug has a pronounced effect when administered by inhalation. Other surviving animals that received Dalargin as a therapeutic/preventive regimen exhibited very mild pathomorphological signs. In groups 8 and 9, in which Dalargin was used preventively, almost all surviving animals showed no signs of pathology and did not exhibit the characteristic histological features of infection.

Therefore, maximum antiviral activity was demonstrated for Dalargin inhalation, whose protective effect with respect to lung tissue and lung function was superior to that of Tamiflu and Kagocel. Dalargin administered by inhalation resulted in the lowest animal mortality and minimized lung tissue damage owing to the immunomodulating effects of the drug and enhanced cellular resistance to viral infection. With preventive administration of Dalargin, surviving animals were not infected, and the phenomenon of antiviral protection was due to the effective prophylactic action of Dalargin in the form of direct antiviral effect and activation of local cell-mediated immunity at the "portal of entry": the nose, nasopharynx, oropharynx and the oral cavity, and in the case of inhalation, additionally the bronchi and trachea. The efficacy attributed exclusively to the prophylactic protective effect without the therapeutic component, is 50 %.

Survival analysis for different doses of Dalargin demonstrated that survival rate increases with dose up to a certain maximum value, which corresponds to a sigmoid dose-response curve.

**Example 7. In vivo evaluation of the antiviral activity of Dalargin administered as a therapeutic or preventive regimen in a model of influenza pneumonia in mice with respect to a pandemic strain of influenza virus A (H1N1) pdm09 (A/California/04/2009).**

Materials and Methods. A previous study of the antiviral activity of Dalargin in a model of influenza pneumonia in mice demonstrated that intranasal administration is non-inferior to intramuscular administration. Meanwhile, the group that received the drug by a combination of intranasal administration and application of spray to the posterior pharyngeal wall (PS) exhibited better survival statistics than the group receiving the drug exclusively via the intranasal route. The maximum effect in this study was observed for the dosage range 0.2-0.5 mg/kg and concentration range 0.08-0.1 % respectively. [17]. These results prompted the next study, which was based on the assumption that the inhalation route of administration can enhance the drug's efficacy and emerge as the preferred target of development.

A model of administration by inhalation was based on the calculations provided in Example 6.

Strain AICalifornia/04/2009, which was first isolated in the USA in 2009 and adapted to mice, was provided by the A.A. Smorodintsev Influenza Research Institute of the Ministry of Health of Russia. Ingavirin® and Arbidol® were used as comparator drugs (active control). The comparator drugs were administered intragastrically at doses of 0.2 mL according to the therapeutic regimen (at 24, 48, 72, 96 and 120 h after challenge).

The animals were randomized across 10 groups of 33 animals each (30 animals for mortality evaluation and 3 animals to be euthanised on day 5) (for a total of n = 330):

group 1 (control): inhalation of saline solution twice a day for 6 days (at 24 h and 1 h before challenge and for 5 days after challenge);

Group 2 (Ingavirin® at a dose of 15 mg/kg intragastrically once a day);

Group 3 (Arbidol® at a dose of 130 mg/kg intragastrically once a day);

group 4 (Dalargin nasally once a day at a dose of 0.2 mg/kg, concentration 0.08 %, 10 μL per route of administration)

Group 5 (Dalargin, nasally and applied to the posterior pharyngeal wall (PS) once daily at a dose of 0.5 mg/kg, concentration 0.1 %, 10 μL per route of administration)

Group 6 (inhalation of 1 % Dalargin solution for 40 minutes once a day). The dose received (the dose that reached the alveolae) was 0.5 mg/kg. The HED corresponds to 3 mg/kg;

Group 7: the mice received 3 % Dalargin solution by inhalation for 45 minutes once a day. The received dose (the dose that reached the alveolae) was 1.74 mg/kg, which corresponds to an HED of 10 mg/kg.

Group 8: the mice received a prophylactic regimen of Ingavirin® at a dose of 15 mg/kg intragastrically once a day on days 3, 2, 1 and at 1 hour before challenge.

Group 9: the mice received inhalations of 3 % Dalargin solution for 45 minutes twice a day on days 3, 2 and 1 and at 1 hour before challenge; no further treatment was administered after the challenge.

Group 10: the product was administered nasally and applied by spraying on the posterior pharyngeal wall (PS) at a dose of 0.5 mg/kg (as 5 μL of 0.2 % solution and 10 μL of 0.1 % solution) once a day on days 3, 2 and 1 and at 1 hour before challenge; no treatment was administered after the challenge.

The following parameters were used as primary efficacy endpoints:

Mortality (%): ratio of the number of dead animals relative to the total number of infected animals in the group: M% = M / Nt, where M is the number of dead animals, and Nt is the number of infected animals.

Protective index (%): ratio of the difference in mortality percentage in the control and experimental groups to mortality percentage in the control group: PI = ((Me - Me) / Me) × 100 %, where Me is the control group mortality, and Me is the experimental group mortality.

Survival was evaluated using the Mantel-Cox method adjusted for multiple comparisons, Kaplan-Meier curves were plotted using IBM SPSS v. 22.0.

Determination of viral load in pulmonary tissue.
The animals were challenged under ether anaesthesia. Mice were challenged with 2.0 lg TCID□□ per animal delivered as 0.025 mL of virus-containing material per nostril.
Three animals per group were euthanised for viral load determinations. In order to determine viral load, the lungs of the mice were harvested on day 5 after challenge and homogenized in a tenfold excess of sterile phosphate buffer, and pooled homogenates of each group were used to prepare a ten-fold serial dilution in the same buffer. This serial dilutions was used to infect MDCK cells in 96-well microplates, which were cultivated for 48 hours at 36 °C in an atmosphere with 5 % COD. Viral load was calculated using the Spearman-Karber method [18] and was expressed as lg TCID□□/mL (lg of the number of 50% tissue culture infective doses per mL).

Results

Clinical signs of disease in infected mice were typical of influenza infections. There was no non-specific mortality in the intact group. Data on mortality dynamics are summarized in Table 3.
Three animals from each group were euthanised on day 5 of the experiment in order to determine viral load in lung tissue. Viral load test results are plotted in Fig. 1 in lg TCID□□/mL.
The results indicate that Dalargin administered by inhalation is superior to other antiviral drugs (Ingavirin® and Arbidol®) for the prevention of influenza pneumonia. Moreover, it was demonstrated that increased efficacy could not be achieved by increasing the inhaled doses of Dalargin. The maximum efficacy was demonstrated for group 6, which received 1 % Dalargin solution by inhalation for 40 minutes once daily. The single absorbed dose (the dose that reached the alveolae) was 0.5 mg/kg, which corresponds to an HED of 3 mg/kg. These data correlated with the viral load determined in the lungs (Fig. 1).
It was also shown that preventive administration of Dalargin exhibits a protective effect against infection in 50-70% of the animals depending on the route of administration. The phenomenon of antiviral protection was due to the pronounced prophylactic effect of Dalargin manifesting as direct antiviral effect and activation of local cell-mediated immunity at the "portal of entry": the nose, nasopharynx, oropharynx and the oral cavity, and in the case of inhalation, additionally the bronchi and trachea.

**Example 8. Clinical case**

Patient K, age 35 years. The patient presented to the hospital unassisted, with symptoms of dry cough, severe fatigue, sweating, dizziness, fever up to 39 °C.

Diagnosis at admission: VRI.

Medical history. Comorbidities: type 2 diabetes mellitus; class III obesity. Acute onset, self-treatment with plenty of fluid, honey, bed rest. The condition did not improve, whereupon the patient, unassisted, sought medical attention at the hospital.

Symptoms: fever for the previous 4 days, muscle and joint ache, pain on eye movement, photophobia, dry cough with scanty mucous sputum, growing weakness, difficulty breathing, retrosternal ache and pains. Did not receive influenza vaccinations for the previous 5 years.

At admission: the condition is moderately severe. Body temperature: 38.6 °C. Pale skin. Harsh bronchial breath sounds on auscultation. RR at rest: 20/min.; BP: 130/90 mm Hg; HR: 90/min. SaO□ = 91%. The abdomen is soft, nontender, without oedema. Swabs for PCR and virology were collected from the nasopharynx and oropharynx. Sputum was collected for bacteriology.

Radiology findings: symmetrical uniform prominence of pulmonary vascularity, the contours are sharp, the roots are structured. Fine-focal shadows at the periphery of the lower pulmonary lobes. The radiological pattern is consistent with stage I of acute respiratory distress syndrome (ARDS).

ECG: Sinus rhythm. Horizontal position of the CA. HR: 94 bpm. Weak amplitude of the P wave is observed in leads I-II and chest leads V2-V6.

Laboratory and instrumental test results: complete blood count: haemoglobin: 116 g/L, RBC: $4.2 \cdot 10^{12}$/L, WBC: $4,1 \cdot 10$□/L, eosinophils: 0 %, basophils: 0%, band cells 5.0%; segmented cells : 52,0%, lymphocytes: 42.0 %, monocytes: 1.0 %, ESR: 13 mm/hr; clinical urinalysis: sp. gravity: 1012, protein: 25, glucose: positive, epithelium: considerable, WBC: 2-10 per FOV, RBC: 1-2 per FOV.

PCR express testing: influenza virus A detected.

Diagnosis (ICD-10: J10.0): moderate influenza complicated by pneumonia, influenza virus identified. Influenza pneumonia.

Therapy administered. Primary therapy: Oseltamivir: 75 mg twice a day for 5 days; additionally: inhalation of 0.02 % Dalargin solution twice a day using a nebulizer, total dose 1 mg/day. Symptomatic therapy: antipyretic, detoxification and mucolytic therapy. Adjuvant therapy with hypoglycaemic drugs. Blood glucose monitoring. The start of Dalargin inhalation procedures was immediately followed by positive changes in the patient's condition. Notable improvements included a reduction of clinical signs of intoxication, onset of conversion of dry cough to productive cough, return of appetite on day 2. Symptoms have completely resolved by day 7 after the start of therapy.

Virology detected strain A/Aichi/2/68 (H3N2).

Sputum bacteriology detected no bacterial contamination as a causative factor of pneumonia. There was consequently no need to administer additional antibacterial treatment.

Condition at discharge: satisfactory. Clinical and laboratory parameters: within the normal range. ECG shows normalization of the shape and size of the P wave. Radiology: lung fields exhibit no focal or infiltrative shadows; pulmonary vascularity is not increased, the roots are structured, not dilated; chest organs show no signs of residual fibrotic changes. Spirometry results show no FEV or FVC deviations.

**Example 9. Clinical case. Use of Dalargin as an agent for the prevention of influenza infection**

In order to avoid contracting influenza, relatives of patient V. (4 persons) self-administered Dalargin from day 1 of the onset of symptoms in the primary patient according to a similar regimen, but once a day (0.02 % solution for inhalation once a day administered with a nebulizer at a dose of 1 mg/day, the total volume of the inhalation solution was 5 mL).

The family members of the patient had not received vaccination for the previous 5 years.

A prophylactic regimen of Oseltamivir was not administered, despite having been recommended by the attending physician.

All the family members (a 35-year-old woman, a 13-year-old child, a 62-year-old woman and a 67-year-old man) had a high risk of contact transmission of influenza infection within the household.

Dalargin was administered by inhalation as 0.02 % solution once daily at a dose of 1 mg/day (with a nebulizer) as a prophylactic treatment.

None of the relatives of patient V who were in contact with him exhibited catarrhal signs, hyperthermia or other signs or symptoms of influenza throughout the possible incubation period: 7 days from the onset of disease and symptoms in patient V.

Conclusion: inhalation of Dalargin solution is an effective method of influenza prophylaxis in persons at high risk of disease transmission. This product can be used as monotherapy or as part of a combination prophylactic regimen with other antiviral drugs. This finding may justify a clinical trial of the efficacy of Dalargin inhalation for the prevention of influenza and other VRIs.

**Example 10. Clinical case: use of Dalargin in moderate uncomplicated influenza on an outpatient basis.**

Patient V, age 37 years. The patient called an ambulance due to the acute onset of the disease. The patient's condition is moderate. Body temperature: 39.1 °C. Severe infectious toxicosis; headache, weakness, hyperhidrosis, joint and muscle ache. Catarrh: dry troublesome cough with retrosternal pain, sore throat and runny nose. Auscultation reveals harsh bronchial breath sounds that are not weakened in the lower lobes. RR at rest: 22/min.; BP: 110/75 mm Hg; HR: 92/min. SaO□ = 92%. No meningeal syndrome. The abdomen is soft, nontender, without oedema.. Did not receive influenza vaccination for the previous 5 years.

Diagnosis made by the ambulance team: VRI (influenza ?). Antipyretic therapy administered. The patient was left under the supervision of the local physician.

Swabs for virology were collected from the nasopharynx and oropharynx. Antiviral therapy prescribed: Oseltamivir 75 mg twice a day for 5 days. Additional recommendations included symptomatic antipyretic and mucolytic therapy, bed rest and drinking plenty of fluid.

The patient self-administered 0.1 % Dalargin solution twice a day by inhalation using a nebulizer, the total dose was 4 mg/day. Clinical signs of intoxication rapidly improved after the start of inhalations. On day 2 of therapy dry cough turned to productive cough, and appetite improved. Fever ended on day 3 of therapy. Symptoms have completely resolved by day 5 after the onset of the disease (and, correspondingly, start of Dalargin therapy).

Virology results detected strain A/California/07/2009 (H1N1, pandemic strain).

Diagnosis (ICD-10: J10.0): moderate influenza, influenza virus identified.

Conclusion: the average duration of moderate influenza is 6-8 days, the usual duration of fever (in case of uncomplicated influenza) is approximately 5 days. {25}. However, in this clinical case, additional treatment with Dalargin by inhalation was associated with a significant reduction in the duration of intoxication symptoms (2 days), fever (3 days) and the disease as a whole (5 days).

The existing clinical presentation of severe complications of VRIs, including severe forms of influenza and coronavirus infections, indicates that the severe stage of the diseases accounts for over 90% of treatment costs and the vast majority of patient fatalities. Treatment at the stage of pneumonia complications is a rather expensive and inefficient process.

Therefore, the optimum way to reduce mortality and costs at the national level would involve administering Dalargin either in spray form to irrigate the nasal passages, nasopharynx, oropharynx and the oral cavity or by inhalation to people from at-risk populations: those living with infected family members, those in contact with patients or large groups of people, such as law enforcement officers, army servicemen, etc. Whenever an infection occurs, administering Dalargin solutions by inhalation according to the regimens described would highly likely allow to avoid complications and keep the disease mild, even avoid hospitalisation.

References

[1] V.I. Pokrovsky et al. Influenza and influenza-like infections (including especially dangerous forms of influenza infections) // Basic and applied aspects of study: Bulletin of the task group. St Petersburg, Roza mira. - 2008. - Vol. 109.

[2] N.B. Lazareva, M.V. Zhuravlyova, L.R. Panteleyeva. ARVI: Rational Pharmacotherapy from the Standpoint of Clinical Pharmacology // Meditsinsky Sovet. - 2016. - № 4..

[3] Sullivan K. M., Monto A. S., Longini Jr I. M. Estimates of the US health impact of influenza //American journal of public health. - 1993. - Vol. 83. - № 12. - p. 1712-1716.

[4] According to reports of the Federal State Healthcare Institution "Federal Centre for Hygiene and Epidemiology of Rospotrebnadzor of the Russian Federation". URL: Available at: http://www.fcgsen.ru.

[5] Clinical Practice Guidelines on Severe Forms of Influenza, 2016, Ministry of Health of Russia, ICD-10: J10-J11.

[6] Turner R. B. Epidemiology, pathogenesis, and treatment of the common cold //Annals of allergy, asthma & immunology. - 1997. - Vol. 78. - №. 6. - p. 531-540..

[7] Epidemiology, Clinical Presentation and Treatment of Severe Forms of Combined Viral-Bacterial Pneumonias. Moscow Oblast Clinical Research Institute, textbook, Moscow, 2016.

[8] O.A. Loskutov, A.N. Druzhina, V.G. Kolesnikov. Antiviral and antibacterial therapy in the treatment of community-acquired pneumonias/ // Meditsina Neotlozhnykh Sostoyaniy. - 2016. - №. 3 (74)..

[9] URL: https://lor-bolezni.ru/pnevmoniya/ingalyatsii-pri-pnevmonii-nebulajzerom.html, accessed on 02.07.2019.

[10] T.F. Borovskaya, E.Kh. Kurpas, S.N. Gorislavets. Application of Dalargin for the treatment of patients with community-acquired pneumonia // Byulleten Fiziologii i Patologii Dykhaniya. - 2004. - №. 17..

[11] V.K. Khugayeva. Effect of Dalargin on microhemocirculation and lymphatic microcirculation // Byul. eksper. biol. - 1998. - №. 3. - p. 300-302..

[12] G.V. Lisachenko. Effect of Dalargin on haemodynamics in acute myocardial infarction complicated by clinical death / G.V. Lisachenko> G.K. Zoloyev, V.D. Slepushkin // Anest. i reanimat. - 1992. - №. 4. - p. 57-59..

[13] V.P. Dubynina. Nebulizer therapy of acute and chronic respiratory tract diseases // Moscow, Inter-Eton, 2008. - 48 pages - 2005 (

[14] Gur D. et al. Inhalational gentamicin treatment is effective against pneumonic plague in a mouse model //Frontiers in microbiology. - 2018. - Vol. 9. -p. 741..

[15] Méndez L. B., Gookin G., Phalen R. F. Inhaled aerosol particle dosimetry in mice: a review //Inhalation toxicology. - 2010. - Vol. 22. - №. 12. - p. 1032-1037..

[16] Kolanjiyil A. V. et al. Mice-to-men comparison of inhaled drug-aerosol deposition and clearance //Respiratory physiology & neurobiology. - 2019. - Vol. 260. - p. 82-94..

[17] R&D Report "Evaluation of the antiviral activity of Orvidal API with nasal and intramuscular administration at different doses and concentrations in an influenza pneumonia model in mice". IFOR. Tomsk, 2020.

[18] Virology. A practical approach / Translated from English B. W. J. Mahy. 1988; 344 pages.

[19] V. A. Aleksandrovskaya et al. Dalargin: Pharmacological and clinical aspects. // Pediatriya, 1993 - No. 3 - pp. 101-104.

[20] E.I. Kalenikova, O.F. Dmitriyeva, N.V. Korobov, S.V. Zhukovsky, V.A. Tishchenko, V.V. Vinogradov. Pharmacokinetis of Dalargin // Voprosy Med. Khimii, 1988, No. 1, pp. 75-83.

[21] Kotzer C.J., Hay D.W., Dondio G., Giardina G., Petrillo P., Underwood D.C. The antitussive activity of delta-opioid receptor stimulation in guinea pigs. J Pharmacol Exp Ther 2000; 292(2): 803-809..

[22] P. B. M. C. N. P. M. V. P. P. M. N. o. f. t. p. o. d. i. t. i. p. A. J. o. H.-S. P. V. 7. I. 1. 1. J. 2. P. Titilola M. Afolabi.

[23] Patent RU 2672888 dated 18.04.2018. Antiviral Immunotropic Agent for the Treatment of Acute Respiratory Viral Infections.

[24] Ansaldi, F., Banfi, F., Morelli, P., Valle, L., Durando, P., Sticchi, L., Contos, S., Gasparini, R., & Crovari, P. (2004). SARS-CoV, influenza A and syncitial respiratory virus resistance against common disinfectants and ultraviolet irradiation. Journal of Preventive Medicine and Hygiene, 45(1-2), 5-8.

[25] Clinical Practice Recommendations "Influenza in Adults". Non-profit partnership "National Scientific Society of Infectologists". Approved on 30 October 2014.

Table 1

| Treatment group / test substance | Concentration, $\mu$g/mL | Virus inhibiting activity parameters | | | | | | | |
|---|---|---|---|---|---|---|---|---|---|
| | | PR8 (influenza virus) | | | | Ad5 (adenovirus) | | | |
| | | Virus titer (lg TCID$\square\square$/ 0.1 mL) | CCD$\square$, $\mu$g/mL | IC$\square$, $\mu$g/mL | SI | Virus titer (lg TCID$\square\square$/0.1 mL) | CCD$\square$, $\mu$g/mL | IC$\square\square$, $\mu$g/m L | SI |
| Dalargin | 30 | 4,0 | | | | 3,5 | | | |
| Dalargin | 10 | 4,5 | | | | 4,0 | | | |
| Dalargin | 3,3 | 5,0 | 30 | 0,8 | >37,5 | 4,0 | 30 | 2,5 | 12 |
| Dalargin | 1,1 | 5,0 | | | | 4,5 | | | |
| Dalargin | 0,4 | 5,5 | | | | 4,5 | | | |
| Virus control | 0 | 5,5 | | | | 4,5 | | | |
| Remantadi n comparator | 5 $\mu$g/mL | - | 64 | 11 | 5 | | | | |
| Aciclovir comparator | 30 $\mu$g/mL | | | | | - | > 30 | > 30 | 1 |

Table 2

| Animal group | Animals per group | Mortality by day | | | | | | | Residue | Mortality , % | Protecti ve index, % |
|---|---|---|---|---|---|---|---|---|---|---|---|
| | | 5 | 6 | 7 | 8 | 9 | 10 | 11 | | | |
| 1 | 30 | 3 | 4 | 7 | 7 | 5 | - | - | 4 | 86,67 | - |
| 2 | 30 | - | - | - | - | 2 | 1 | - | 27* | 10,00 | 88,46 |
| 3 | 30 | 2 | 2 | 5 | 4 | 3 | 1 | - | 13 | 56,67 | 34,61 |
| 4 | 30 | - | - | - | - | 2 | - | 1 | 27* | 10,00 | 88,46 |
| 5 | 30 | - | - | - | 1 | - | 1 | - | 28* | 6,67 | 92,30 |
| 6 | 30 | - | - | 1 | - | - | 1 | - | 28* | 6,67 | 92,30 |
| 7 | 30 | 2 | 3 | 6 | 6 | 4 | 1 | 1 | 7 | 76,67 | 11,54 |
| 8 | 30 | 1 | 3 | 2 | 3 | 3 | - | - | 18 | 40,00 | 53,85 |
| 9 | 30 | - | 4 | 4 | 4 | 2 | 1 | - | 15 | 50,00 | 42,31 |

Table 3

| Animal group | Animals per group | Mortality by day | | | | | | | Residue | Mortality , % | Protecti ve index, % |
|---|---|---|---|---|---|---|---|---|---|---|---|
| | | 5 | 6 | 7 | 8 | 9 | 10 | 11 | | | |
| 1 | 30 | 3 | 5 | 6 | 7 | 5 | 2 | 1 | 1 | 96,67% | - |
| 2 | 30 | - | 4 | 6 | 3 | 2 | 1 | - | 14 | 53,33% | 44,83% |
| 3 | 30 | 2 | 2 | 7 | 5 | 3 | 2 | - | 9 | 70,00% | 27,59% |
| 4 | 30 | 2 | 4 | 9 | - | 1 | - | 1 | 13 | 56,67% | 41,38% |
| 5 | 30 | - | 1 | 2 | 3 | 5 | 1 | - | 18 | 40,00% | 58,62% |
| 6 | 30 | - | - | 1 | 1 | - | - | - | 28 | 6,67%** | 93,10% |
| 7 | 30 | - | - | 1 | 1 | 1 | - | - | 27 | 10,00%* * | 89,66% |
| 8 | 30 | 3 | 4 | 4 | 7 | 4 | 2 | - | 6 | 80,00% | 17,24% |
| 9 | 30 | - | 2 | 3 | 2 | 2 | - | - | 21 | 30,00% | 68,97% |
| 10 | 30 | 2 | 4 | 2 | 3 | 2 | 1 | - | 16 | 46,67% | 51,72% |

**Claims**

1. Tyrosyl-D-alanyl-glycyl-phenylalanyl-leucyl-arginine diacetate for use in spray form and/or in an inhaled form for the prevention of viral respiratory infections or for the prevention of their complications by administration in spray form to irrigate the nasal passages, nasopharynx, oropharynx and the oral cavity and/or by inhalation.

2. Tyrosyl-D-alanyl-glycyl-phenylalanyl-leucyl-arginine diacetate according to claim 1, as a component of a composition that is administered as a spray and/or as an inhalation solution.

3. Tyrosyl-D-alanyl-glycyl-phenylalanyl-leucyl-arginine diacetate for use in the composition according to claim 2, wherein the composition is used for the prevention of viral respiratory infections via inhalation or as a spray with a particle size of 5-50 $\mu$m.

4. Tyrosyl-D-alanyl-glycyl-phenylalanyl-leucyl-arginine diacetate for use in the composition according to claim 2, wherein the composition is used for irrigating the entire surface of the nasopharynx, oropharynx, oral cavity and is applied sublingually, under the tongue, to create a protective barrier effect on mucous membranes.

5. Tyrosyl-D-alanyl-glycyl-phenylalanyl-leucyl-arginine diacetate for use in the composition according to claim 2, wherein the composition is used for the prevention of the development of viral respiratory infection complications via inhalation with a particle size of 0.5-10 $\mu$m, preferably 0.5-3 $\mu$m.

6. Tyrosyl-D-alanyl-glycyl-phenylalanyl-leucyl-arginine diacetate for use in the composition according to claim 2, wherein the composition, which is administered in spray form and/or as an inhalation solution, contains a microbial growth inhibitor, such as benzalkonium chloride, usually in the concentration range of 0.005-0.1 wt %, preferably 0.01%.

7. Tyrosyl-D-alanyl-glycyl-phenylalanyl-leucyl-arginine diacetate for use in the composition according to claims 2 or 6, wherein the composition has an acidic pH in the range from 3 to 7, preferably pH = 4.0-5.5.

8. Tyrosyl-D-alanyl-glycyl-phenylalanyl-leucyl-arginine diacetate for use in the composition according to claim 7, wherein the composition containing tyrosyl-D-alanyl-glycyl-phenylalanyl-leucyl-arginine diacetate, which is administered in spray form and/or as an inhalation solution, contains acetate buffer.

9. Tyrosyl-D-alanyl-glycyl-phenylalanyl-leucyl-arginine diacetate for use in the composition according to claims 2 to 8, containing viscosity regulator selected from the group of microcrystalline cellulose, sodium carboxymethyl cellulose, macrogol, propylene glycol, glycerol, polyvinylpyrrolidone and N-methylpyrrolidone and/or polymers with bioadhesive properties, surfactants, or non-ionic detergents with mucoadhesive properties, preferably poloxamers.

10. Tyrosyl-D-alanyl-glycyl-phenylalanyl-leucyl-arginine diacetate for use in the composition according to claims 2 to 9, wherein the administration in spray form comprises simultaneous irrigation of the oral cavity, nasopharynx, oropharynx and the sublingual area.

**Patentansprüche**

1. Tyrosyl-O-alanyl-glycyl-phenylalanyl-leucyl-arginin-diacetat zur Verwendung in Sprayform und/oder in inhalierter Form zur Vorbeugung viraler Atemwegsinfektionen oder zur Prävention ihrer Komplikationen durch Verabreichung in Sprayform zum Spülen der Nasengänge, des Nasopharynx, des Oropharynx und der Mundhöhle und/oder durch Inhalation.

2. Tyrosyl-O-alanyl-glycyl-phenylalanyl-leucyl-arginin-diacetat nach Anspruch 1, als Komponente einer Zusammensetzung, die als Spray und/oder als Inhalationslösung verabreicht wird.

3. Tyrosyl-O-alanyl-glycyl-phenylalanyl-leucyl-arginin-diacetat zur Verwendung in der Zusammensetzung nach Anspruch 2, wobei die Zusammensetzung zur Prävention viraler Atemwegsinfektionen durch Inhalation oder als Spray mit einer Teilchengröße von 5-50 $\mu$m verwendet wird.

4. Tyrosyl-O-alanyl-glycyl-phenylalanyl-leucyl-arginin-diacetat zur Verwendung in der Zusammensetzung nach Anspruch 2, wobei die Zusammensetzung zum Spülen der gesamten Oberfläche des Nasopharynx, des Oropharynx, der Mundhöhle verwendet wird und sublingual, unter der Zunge, angewendet wird, um eine schützende Barrierewirkung auf die Schleimhäute zu schaffen.

5. Tyrosyl-O-alanyl-glycyl-phenylalanyl-leucyl-arginin-diacetat zur Verwendung in der Zusammensetzung nach Anspruch 2, wobei die Zusammensetzung zur Prävention der Entwicklung von Komplikationen viraler Atemwegsinfektionen durch Inhalation mit einer Teilchengröße von 0,5-10 $\mu$m, vorzugsweise 0,5-3 $\mu$m, verwendet wird.

6. Tyrosyl-O-alanyl-glycyl-phenylalanyl-leucyl-arginin-diacetat zur Verwendung in der Zusammensetzung nach Anspruch 2, wobei die Zusammensetzung, die in Sprayform und/oder als Inhalationslösung verabreicht wird, einen mikrobiellen Wachstumshemmer, wie beispielsweise Benzalkoniumchlorid, üblicherweise in dem Konzentrationsbereich von 0,005-0,1 Gewichts-%, vorzugsweise 0,01 %, enthält.

7. Tyrosyl-O-alanyl-glycyl-phenylalanyl-leucyl-arginin-diacetat zur Verwendung in der Zusammensetzung nach Anspruch 2 oder 6, wobei die Zusammensetzung einen sauren pH in dem Bereich von 3 bis 7, vorzugsweise pH = 4,0-5,5, aufweist.

8. Tyrosyl-O-alanyl-glycyl-phenylalanyl-leucyl-arginin-diacetat zur Verwendung in der Zusammensetzung nach Anspruch 7, wobei die Zusammensetzung, die Tyrosyl-O-alanyl-glycyl-phenylalanyl-leucyl-arginin-diacetat enthält, die in Sprayform und/oder als Inhalationslösung verabreicht wird, Acetatpuffer enthält.

9. Tyrosyl-O-alanyl-glycyl-phenylalanyl-leucyl-arginin-diacetat zur Verwendung in der Zusammensetzung nach einem der Ansprüche 2 bis 8, die einen Viskositätsregulator enthält, ausgewählt aus der Gruppe von mikrokristalliner Cellulose, Natriumcarboxymethylcellulose, Macrogol, Propylenglykol, Glycerin, Polyvinylpyrrolidon und N-Methylpyrrolidon und/oder Polymeren mit bioadhäsiven Eigenschaften, Tensiden oder nichtionischen Detergenzien mit mukoadhäsiven Eigenschaften, vorzugsweise Poloxameren.

10. Tyrosyl-O-alanyl-glycyl-phenylalanyl-leucyl-arginin-diacetat zur Verwendung in der Zusammensetzung nach einem der Ansprüche 2 bis 9, wobei die Verabreichung in Sprayform eine gleichzeitige Spülung der Mundhöhle, des Nasopharynx, des Oropharynx und des sublingualen Bereichs umfasst.

**Revendications**

1. Diacétate de tyrosyl-D-alanyl-glycyl-phénylalanyl-leucyl-arginine destiné à être utilisé sous forme d'aérosol et/ou sous forme inhalée pour la prévention des infections respiratoires virales ou pour la prévention de leurs complications par administration sous forme d'aérosol pour irriguer les voies nasales, le nasopharynx, l'oropharynx et la cavité buccale et/ou par inhalation.

2. Diacétate de tyrosyl-D-alanyl-glycyl-phénylalanyl-leucyl-arginine selon la revendication 1, en tant que composant d'une composition qui est administrée sous forme d'aérosol et/ou sous forme de solution pour inhalation.

3. Diacétate de tyrosyl-D-alanyl-glycyl-phénylalanyl-leucyl-arginine pour utilisation dans la composition selon la revendication 2, la composition étant utilisée pour la prévention des infections respiratoires virales par inhalation ou sous forme d'aérosol avec une taille de particule de 5 à 50 μm.

4. Diacétate de tyrosyl-D-alanyl-glycyl-phénylalanyl-leucyl-arginine pour utilisation dans la composition selon la revendication 2, la composition étant utilisée pour irriguer la surface entière du nasopharynx, de l'oropharynx, de la cavité buccale et étant appliquée par voie sublinguale, sous la langue, pour créer un effet barrière protecteur sur les membranes muqueuses.

5. Diacétate de tyrosyl-D-alanyl-glycyl-phénylalanyl-leucyl-arginine pour utilisation dans la composition selon la revendication 2, la composition étant utilisée pour la prévention du développement de complications d'infections respiratoires virales par inhalation avec une taille de particule de 0,5 à 10 μm, de préférence 0,5 à 3 μm.

6. Diacétate de tyrosyl-D-alanyl-glycyl-phénylalanyl-leucyl-arginine pour utilisation dans la composition selon la revendication 2, la composition, qui est administrée sous forme d'aérosol et/ou sous forme de solution pour inhalation, contenant un inhibiteur de croissance microbienne, tel que le chlorure de benzalkonium, généralement dans la plage de concentration de 0,005 à 0,1 % en poids, de préférence 0,01 %.

7. Diacétate de tyrosyl-D-alanyl-glycyl-phénylalanyl-leucyl-arginine pour utilisation dans la composition selon les revendications 2 ou 6, la composition ayant un pH acide dans la plage de 3 à 7, de préférence pH = 4,0 à 5,5.

8. Diacétate de tyrosyl-D-alanyl-glycyl-phénylalanyl-leucyl-arginine pour utilisation dans la composition selon la revendication 7, la composition contenant du diacétate de tyrosyl-D-alanyl-glycyl-phénylalanyl-leucyl-arginine, qui est administrée sous forme d'aérosol et/ou sous forme de solution pour inhalation, contenant un tampon acétate.

9. Diacétate de tyrosyl-D-alanyl-glycyl-phénylalanyl-leucyl-arginine pour utilisation dans la composition selon les revendications 2 à 8, contenant un régulateur de viscosité choisi dans le groupe de la cellulose microcristalline, de la carboxyméthylcellulose sodique, du macrogol, du propylène glycol, du glycérol, de la polyvinylpyrrolidone et de la N-méthylpyrrolidone et/ou des polymères ayant des propriétés bioadhésives, des tensioactifs ou des détergents non ioniques ayant des propriétés mucoadhésives, de préférence des poloxamères.

10. Diacétate de tyrosyl-D-alanyl-glycyl-phénylalanyl-leucyl-arginine pour utilisation dans la composition selon les revendications 2 à 9, dans lequel l'administration sous forme d'aérosol comprend l'irrigation simultanée de la cavité buccale, du nasopharynx, de l'oropharynx et de la zone sublinguale.

Fig. 1

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- SU 1475670 A1
- RU 2436588 C1
- EP 3556377 A1
- RU 2672888

## Non-patent literature cited in the description

- **OZBEJ ZUPANCIC et al.** Development and in vitro characterization of self-emulsifying drug delivery system (SEDDS) for oral opioid peptide delivery. *JOURNAL DRUG DEVELOPMENT AND INDUSTRIAL PHARMACY,* 04 July 2017, vol. 43 (10), 1694-1702
- **V.I. POKROVSKY et al.** Influenza and influenza-like infections (including especially dangerous forms of influenza infections). *Basic and applied aspects of study: Bulletin of the task group. St Petersburg, Roza mira.,* 2008, vol. 109
- *reports of the Federal State Healthcare Institution "Federal Centre for Hygiene and Epidemiology of Rospotrebnadzor of the Russian Federation, http://www.fcgsen.ru*
- Clinical Practice Guidelines on Severe Forms of Influenza. Ministry of Health of Russia, 2016, J10-J11
- Epidemiology, Clinical Presentation and Treatment of Severe Forms of Combined Viral-Bacterial Pneumonias. Moscow Oblast Clinical Research Institute, 2016
- **V.P. DUBYNINA.** Nebulizer therapy of acute and chronic respiratory tract diseases. *Moscow, Inter-Eton,* 2008, vol. 48, 2005
- **GUR D. et al.** Inhalational gentamicin treatment is effective against pneumonic plague in a mouse model. *Frontiers in microbiology,* 2018, vol. 9, 741
- **KOLANJIYIL A. V. et al.** Mice-to-men comparison of inhaled drug-aerosol deposition and clearance. *Respiratory physiology & neurobiology,* 2019, vol. 260, 82-94
- Evaluation of the antiviral activity of Orvidal API with nasal and intramuscular administration at different doses and concentrations in an influenza pneumonia model in mice. *R&D Report,* 2020
- **B. W. J. MAHY.** *Virology. A practical approach,* 1988, 344
- **V. A. ALEKSANDROVSKAYA et al.** Dalargin: Pharmacological and clinical aspects. *Pediatriya,* 1993, 101-104
- **E.I. KALENIKOVA ; O.F. DMITRIYEVA ; N.V. KOROBOV ; S.V. ZHUKOVSKY ; V.A. TISHCHENKO ; V.V. VINOGRADOV.** Pharmacokinetis of Dalargin. *Voprosy Med. Khimii,* 1988, 75-83
- **KOTZER C.J. ; HAY D.W. ; DONDIO G. ; GIARDINA G. ; PETRILLO P.** Underwood D.C. The antitussive activity of delta-opioid receptor stimulation in guinea pigs. *J Pharmacol Exp Ther,* 2000, vol. 292 (2), 803-809
- **ANSALDI, F. ; BANFI, F. ; MORELLI, P. ; VALLE, L. ; DURANDO, P. ; STICCHI, L. ; CONTOS, S. ; GASPARINI, R. ; CROVARI, P.** SARS-CoV, influenza A and syncitial respiratory virus resistance against common disinfectants and ultraviolet irradiation. *Journal of Preventive Medicine and Hygiene,* 2004, vol. 45 (1-2), 5-8
- Influenza in Adults. Clinical Practice Recommendations. National Scientific Society of Infectologists, 30 October 2014